# EUROPEAN PATENT SPECIFICATION

(11) **EP 1 148 066 B1**
(45) Date of publication and mention of the grant of the patent: **09.01.2008**
(21) Application number: 01109374.7
(22) Date of filing: 18.04.2001
(51) Int. Cl.: C07K 14/705, C12N 15/12, C12N 15/86, G01N 33/569, C12N 15/10, C12N 5/06

(54) **Use of CD34 or a polypeptide derived therefrom as cell-surface marker in gene-transfer**
Verwendung von CD34 oder eines davon abgeleiteten Polypeptids als Oberflächenmarker für Gentransfer
Utilisation de CD34 ou d'un polypeptide derivé comme marqueur de la surface cellulaire pour transfert genique

(30) Priority: 18.04.2000 DE 10019075
(43) Date of publication of application: 24.10.2001
(73) Proprietor: Vision 7 GmbH, 22335 Hamburg (DE)
(72) Inventor: Zander, Axel R., Prof. Dr., 22926 Ahrensbrug (DE)
(74) Representative: UEXKÜLL & STOLBERG

(56) References cited:
- US-A- 5 731 425
- LEONARD J P ET AL: "Retroviral transduction of human hematopoietic progenitor cells using a vector encoding a cell surface marker (LNGFR) to optimize transgene expression and characterize transduced cell populations." BLOOD, vol. 88, no. 10 SUPPL. 1 PART 1-2, 1996, page 433A XP001013590 Thirty-eighth Annual Meeting of the American Society of Hematology;Orlando, Florida, USA; December 6-10, 1996 ISSN: 0006-4971
- GARCIA-HERNANDEZ B ET AL: "Retroviral vector design for gene therapy of cancer: Specific inhibition and tagging of BCR-ABL-p190 cells." MOLECULAR MEDICINE (CAMBRIDGE), vol. 2, no. 1, 1996, pages 125-133, XP001013586 ISSN: 1076-1551
- ROSENZWEIG M ET AL: "Efficient and durable gene marking of hematopoietic progenitor cells in nonhuman primates after nonablative conditioning." BLOOD, vol. 94, no. 7, 1 October 1999 (1999-10-01), pages 2271-2286, XP002175410 ISSN: 0006-4971
- SIMMONS D L ET AL: "MOLECULAR CLONING OF A CDNA ENCODING CD34 A SIALOMUCIN OF HUMAN HEMATOPOIETIC STEM CELLS" JOURNAL OF IMMUNOLOGY, vol. 148, no. 1, 1992, pages 267-271, XP001013589 ISSN: 0022-1767

### SEQUENCE LISTING

<110> Prof. Dr. Axel R. Zander
<120> Use of CD34 or a Polypeptide derived therefrom as Cell Surface/Gene Transfer Marker
<130> P056707
<140>
   <141>
<160> 10
<170> PatentIn Ver. 2.0
<210> 1
   <211> 1122
   <212> DNA
   <213> Homo sapiens
<220>
   <221> CDS
   <222> (1)..(1122)
   <223> CD34 (complete length)
<400> 1
   atg ccg cgg ggc tgg acc gcg ctt tgc ttg ctg agt ttg ctg cct tct      48
   Met Pro Arg Gly Trp Thr Ala Leu Cys Leu Leu Ser Leu Leu Pro Ser
     1               5                  10                  15
   ggg ttc atg agt ctt gac aac aac ggt act gct acc cca gag tta cct      96
   Gly Phe Met Ser Leu Asp Asn Asn Gly Thr Ala Thr Pro Glu Leu Pro
                20                  25                  30
   acc cag gga aca ttt tca aat gtt tct aca aat gta tcc tac caa gaa     144
   Thr Gln Gly Thr Phe Ser Asn Val Ser Thr Asn Val Ser Tyr Gln Glu
            35                  40                  45
   act aca aca cct agt acc ctt gga agt acc agc ctg cac cct gtg tct     192
   Thr Thr Thr Pro Ser Thr Leu Gly Ser Thr Ser Leu His Pro Val Ser
        50                  55                  60
   caa cat ggc aat gag gcc aca aca aac atc aca gaa acg aca gtc aaa     240
   Gln His Gly Asn Glu Ala Thr Thr Asn Ile Thr Glu Thr Thr Val Lys
    65                  70                  75                  80
   ttc aca tct acc tct gtg ata acc tca gtt tat gga aac aca aac tct     288
   Phe Thr Ser Thr Ser Val Ile Thr Ser Val Tyr Gly Asn Thr Asn Ser
                    85                  90                  95
   tct gtc cag tca cag acc tct gta atc agc aca gtg ttc acc acc cca     336
   Ser Val Gln Ser Gln Thr Ser Val Ile Ser Thr Val Phe Thr Thr Pro
               100                 105                 110
   gcc aac gtt tca act cca gag aca acc ttg aag cct agc ctg tca cct     384
   Ala Asn Val Ser Thr Pro Glu Thr Thr Leu Lys Pro Ser Leu Ser Pro
           115                 120                 125
   gga aat gtt tca gac ctt tca acc act agc act agc ctt gca aca tct 432
   Gly Asn Val Ser Asp Leu Ser Thr Thr Ser Thr Ser Leu Ala Thr Ser
   130 135 140
   ccc act aaa ccc tat aca tca tct tct cct atc cta agt gac atc aag 480
   Pro Thr Lys Pro Tyr Thr Ser Ser Ser Pro Ile Leu Ser Asp Ile Lys
   145 150 155 160
   gca gaa atc aaa tgt tca ggc atc aga gaa gtg aaa ttg act cag ggc 528
   Ala Glu Ile Lys Cys Ser Gly Ile Arg Glu Val Lys Leu Thr Gln Gly
   165 170 175
   atc tgc ctg gag caa aat aag acc tcc agc tgt gcg gag ttt aag aag 576
   Ile Cys Leu Glu Gln Asn Lys Thr Ser Ser Cys Ala Glu Phe Lys Lys
   180 185 190
   gac agg gga gag ggc ctg gcc cga gtg ctg tgt ggg gag gag cag gct 624
   Asp Arg Gly Glu Gly Leu Ala Arg Val Leu Cys Gly Glu Glu Gln Ala
   195 200 205
   gat gct gat gct ggg gcc cag gta tgc tcc ctg ctc ctt gcc cag tct 672
   Asp Ala Asp Ala Gly Ala Gln Val Cys Ser Leu Leu Leu Ala Gln Ser
   210 215 220
   gag gtg agg cct cag tgt cta ctg ctg gtc ttg gcc aac aga aca gaa 720
   Glu Val Arg Pro Gln Cys Leu Leu Leu Val Leu Ala Asn Arg Thr Glu
   225 230 235 240
   att tcc agc aaa ctc caa ctt atg aaa aag cac caa tct gac ctg aaa 768
   Ile Ser Ser Lys Leu Gln Leu Met Lys Lys His Gln Ser Asp Leu Lys
   245 250 255
   aag ctg ggg atc cta gat ttc act gag caa gat gtt gca agc cac cag 816
   Lys Leu Gly Ile Leu Asp Phe Thr Glu Gln Asp Val Ala Ser His Gln
   260 265 270
   agc tat tcc caa aag acc ctg att gca ctg gtc acc tcg gga gcc ctg 864
   Ser Tyr Ser Gln Lys Thr Leu Ile Ala Leu Val Thr Ser Gly Ala Leu
   275 280 285
   ctg gct gtc ttg ggc atc act ggc tat ttc ctg atg aat cgc cgc agc 912
   Leu Ala Val Leu Gly Ile Thr Gly Tyr Phe Leu Met Asn Arg Arg Ser
   290 295 300
   tgg agc ccc aca gga gaa agg ctg ggc gaa gac cct tat tac acg gaa 960
   Trp Ser Pro Thr Gly Glu Arg Leu Gly Glu Asp Pro Tyr Tyr Thr Glu
   305 310 315 320
   aac ggt gga ggc cag ggc tat agc tca gga cct ggg acc tcc cct gag 1008
   Asn Gly Gly Gly Gln Gly Tyr Ser Ser Gly Pro Gly Thr Ser Pro Glu
   325 330 335
   gct cag gga aag gcc agt gtg aac cga ggg gct cag gaa aac ggg acc 1056
   Ala Gln Gly Lys Ala Ser Val Asn Arg Gly Ala Gln Glu Asn Gly Thr
   340 345 350
   ggc cag gcc acc tcc aga aac ggc cat tca gca aga caa cac gtg gtg 1104
   Gly Gln Ala Thr Ser Arg Asn Gly His Ser Ala Arg Gln His Val Val
   355 360 365
   gct gat acc gaa ttg tga 1122
   Ala Asp Thr Glu Leu
   370
<210> 2
   <211> 373
   <212> PRT
   <213> Homo sapiens
<400> 2
   Met Pro Arg Gly Trp Thr Ala Leu Cys Leu Leu Ser Leu Leu Pro Ser
    1               5                  10                  15
   Gly Phe Met Ser Leu Asp Asn Asn Gly Thr Ala Thr Pro Glu Leu Pro
               20                  25                  30
   Thr Gln Gly Thr Phe Ser Asn Val Ser Thr Asn Val Ser Tyr Gln Glu
           35                  40                  45
   Thr Thr Thr Pro Ser Thr Leu Gly Ser Thr Ser Leu His Pro Val Ser
       50                  55                  60
   Gln His Gly Asn Glu Ala Thr Thr Asn Ile Thr Glu Thr Thr Val Lys
   65                  70                  75                  80
   Phe Thr Ser Thr Ser Val Ile Thr Ser Val Tyr Gly Asn Thr Asn Ser
                   85                  90                  95
   Ser Val Gln Ser Gln Thr Ser Val Ile Ser Thr Val Phe Thr Thr Pro
               100                 105                 110
   Ala Asn Val Ser Thr Pro Glu Thr Thr Leu Lys Pro Ser Leu Ser Pro
           115                 120                 125
   Gly Asn Val Ser Asp Leu Ser Thr Thr Ser Thr Ser Leu Ala Thr Ser
       130                 135                 140
   Pro Thr Lys Pro Tyr Thr Ser Ser Ser Pro Ile Leu Ser Asp Ile Lys
   145                 150                 155                 160
   Ala Glu Ile Lys Cys Ser Gly Ile Arg Glu Val Lys Leu Thr Gln Gly
                   165                 170                 175
   Ile Cys Leu Glu Gln Asn Lys Thr Ser Ser Cys Ala Glu Phe Lys Lys
               180                 185                 190
   Asp Arg Gly Glu Gly Leu Ala Arg Val Leu Cys Gly Glu Glu Gln Ala
           195                 200                 205
   Asp Ala Asp Ala Gly Ala Gln Val Cys Ser Leu Leu Leu Ala Gln Ser
       210                 215                 220
   Glu Val Arg Pro Gln Cys Leu Leu Leu Val Leu Ala Asn Arg Thr Glu
   225                 230                 235                 240
   Ile Ser Ser Lys Leu Gln Leu Met Lys Lys His Gln Ser Asp Leu Lys
                   245                 250                 255
   Lys Leu Gly Ile Leu Asp Phe Thr Glu Gln Asp Val Ala Ser His Gln
               260                 265                 270
   Ser Tyr Ser Gln Lys Thr Leu Ile Ala Leu Val Thr Ser Gly Ala Leu
           275                 280                 285
   Leu Ala Val Leu Gly Ile Thr Gly Tyr Phe Leu Met Asn Arg Arg Ser
       290                 295                 300
   Trp Ser Pro Thr Gly Glu Arg Leu Gly Glu Asp Pro Tyr Tyr Thr Glu
   305                 310                 315                 320
   Asn Gly Gly Gly Gln Gly Tyr Ser Ser Gly Pro Gly Thr Ser Pro Glu
                   325                 330                 335
   Ala Gln Gly Lys Ala Ser Val Asn Arg Gly Ala Gln Glu Asn Gly Thr
               340                 345                 350
   Gly Gln Ala Thr Ser Arg Asn Gly His Ser Ala Arg Gln His Val Val
           355                 360                 365
   Ala Asp Thr Glu Leu
       370
<210> 3
   <211> 951
   <212> DNA
   <213> Homo sapiens
<220>
   <221> CDS
   <222> (1)..(951)
   <223> CD34 (truncated variant)
<400> 3
   atg ccg cgg ggc tgg acc gcg ctt tgc ttg ctg agt ttg ctg cct tct    48
   Met Pro Arg Gly Trp Thr Ala Leu Cys Leu Leu Ser Leu Leu Pro Ser
    1               5                  10                  15
   ggg ttc atg agt ctt gac aac aac ggt act gct acc cca gag tta cct    96
   Gly Phe Met Ser Leu Asp Asn Asn Gly Thr Ala Thr Pro Glu Leu Pro
               20                  25                  30
   acc cag gga aca ttt tca aat gtt tct aca aat gta tcc tac caa gaa   144
   Thr Gln Gly Thr Phe Ser Asn Val Ser Thr Asn Val Ser Tyr Gln Glu
           35                  40                  45
   act aca aca cct agt acc ctt gga agt acc agc ctg cac cct gtg tct   192
   Thr Thr Thr Pro Ser Thr Leu Gly Ser Thr Ser Leu His Pro Val Ser
       50                  55                  60
   caa cat ggc aat gag gcc aca aca aac atc aca gaa acg aca gtc aaa   240
   Gln His Gly Asn Glu Ala Thr Thr Asn Ile Thr Glu Thr Thr Val Lys
   65                  70                  75                  80
   ttc aca tct acc tct gtg ata acc tca gtt tat gga aac aca aac tct   288
   Phe Thr Ser Thr Ser Val Ile Thr Ser Val Tyr Gly Asn Thr Asn Ser
                   85                  90                  95
   tct gtc cag tca cag acc tct gta atc agc aca gtg ttc acc acc cca   336
   Ser Val Gln Ser Gln Thr Ser Val Ile Ser Thr Val Phe Thr Thr Pro
               100                 105                 110
   gcc aac gtt tca act cca gag aca acc ttg aag cct agc ctg tca cct   384
   Ala Asn Val Ser Thr Pro Glu Thr Thr Leu Lys Pro Ser Leu Ser Pro
           115                 120                 125
   gga aat gtt tca gac ctt tca acc act agc act agc ctt gca aca tct   432
   Gly Asn Val Ser Asp Leu Ser Thr Thr Ser Thr Ser Leu Ala Thr Ser
       130                 135                 140
   ccc act aaa ccc tat aca tca tct tct cct atc cta agt gac atc aag   480
   Pro Thr Lys Pro Tyr Thr Ser Ser Ser Pro Ile Leu Ser Asp Ile Lys
   145                 150                 155                 160
   gca gaa atc aaa tgt tca ggc atc aga gaa gtg aaa ttg act cag ggc   528
   Ala Glu Ile Lys Cys Ser Gly Ile Arg Glu Val Lys Leu Thr Gln Gly
                   165                 170                 175
   atc tgc ctg gag caa aat aag acc tcc agc tgt gcg gag ttt aag aag   576
   Ile Cys Leu Glu Gln Asn Lys Thr Ser Ser Cys Ala Glu Phe Lys Lys
               180                 185                 190
   gac agg gga gag ggc ctg gcc cga gtg ctg tgt ggg gag gag cag gct     624
   Asp Arg Gly Glu Gly Leu Ala Arg Val Leu Cys Gly Glu Glu Gln Ala
           195                 200                 205
   gat gct gat gct ggg gcc cag gta tgc tcc ctg ctc ctt gcc cag tct     672
   Asp Ala Asp Ala Gly Ala Gln Val Cys Ser Leu Leu Leu Ala Gln Ser
       210                 215                 220
   gag gtg agg cct cag tgt cta ctg ctg gtc ttg gcc aac aga aca gaa     720
   Glu Val Arg Pro Gln Cys Leu Leu Leu Val Leu Ala Asn Arg Thr Glu
   225                 230                 235                 240
   att tcc agc aaa ctc caa ctt atg aaa aag cac caa tct gac ctg aaa     768
   Ile Ser Ser Lys Leu Gln Leu Met Lys Lys His Gln Ser Asp Leu Lys
                   245                 250                 255
   aag ctg ggg atc cta gat ttc act gag caa gat gtt gca agc cac cag     816
   Lys Leu Gly Ile Leu Asp Phe Thr Glu Gln Asp Val Ala Ser His Gln
               260                 265                 270
   agc tat tcc caa aag acc ctg att gca ctg gtc acc tcg gga gcc ctg     864
   Ser Tyr Ser Gln Lys Thr Leu Ile Ala Leu Val Thr Ser Gly Ala Leu
           275                 280                 285
   ctg gct gtc ttg ggc atc act ggc tat ttc ctg atg aat cgc cgc agc     912
   Leu Ala Val Leu Gly Ile Thr Gly Tyr Phe Leu Met Asn Arg Arg Ser
       290                 295                 300
   tgg agc ccc aca gga gaa agg ctg gaa cta gaa cca tga                 951
   Trp Ser Pro Thr Gly Glu Arg Leu Glu Leu Glu Pro
   305                 310                 315
<210> 4
   <211> 316
   <212> PRT
   <213> Homo sapiens
<400> 4
   Met Pro Arg Gly Trp Thr Ala Leu Cys Leu Leu Ser Leu Leu Pro Ser
   1               5                   10                  15
   Gly Phe Met Ser Leu Asp Asn Asn Gly Thr Ala Thr Pro Glu Leu Pro
               20                  25                  30
   Thr Gln Gly Thr Phe Ser Asn Val Ser Thr Asn Val Ser Tyr Gln Glu
           35                  40                  45
   Thr Thr Thr Pro Ser Thr Leu Gly Ser Thr Ser Leu His Pro Val Ser
       50                  55                  60
   Gln His Gly Asn Glu Ala Thr Thr Asn Ile Thr Glu Thr Thr Val Lys
   65                  70                  75                  80
   Phe Thr Ser Thr Ser Val Ile Thr Ser Val Tyr Gly Asn Thr Asn Ser
                   85                  90                  95
   Ser Val Gln Ser Gln Thr Ser Val Ile Ser Thr Val Phe Thr Thr Pro
               100                 105                 110
   Ala Asn Val Ser Thr Pro Glu Thr Thr Leu Lys Pro Ser Leu Ser Pro
           115                 120                 125
   Gly Asn Val Ser Asp Leu Ser Thr Thr Ser Thr Ser Leu Ala Thr Ser
       130                 135                 140
   Pro Thr Lys Pro Tyr Thr Ser Ser Ser Pro Ile Leu Ser Asp Ile Lys
   145                 150                 155                 160
   Ala Glu Ile Lys Cys Ser Gly Ile Arg Glu Val Lys Leu Thr Gln Gly
                   165                 170                 175
   Ile Cys Leu Glu Gln Asn Lys Thr Ser Ser Cys Ala Glu Phe Lys Lys
               180                 185                 190
   Asp Arg Gly Glu Gly Leu Ala Arg Val Leu Cys Gly Glu Glu Gln Ala
           195                 200                 205
   Asp Ala Asp Ala Gly Ala Gln Val Cys Ser Leu Leu Leu Ala Gln Ser
       210                 215                 220
   Glu Val Arg Pro Gln Cys Leu Leu Leu Val Leu Ala Asn Arg Thr Glu
   225                 230                 235                 240
   Ile Ser Ser Lys Leu Gln Leu Met Lys Lys His Gln Ser Asp Leu Lys
                   245                 250                 255
   Lys Leu Gly Ile Leu Asp Phe Thr Glu Gln Asp Val Ala Ser His Gln
               260                 265                 270
   Ser Tyr Ser Gln Lys Thr Leu Ile Ala Leu Val Thr Ser Gly Ala Leu
           275                 280                 285
   Leu Ala Val Leu Gly Ile Thr Gly Tyr Phe Leu Met Asn Arg Arg Ser
       290                 295                 300
   Trp Ser Pro Thr Gly Glu Arg Leu Glu Leu Glu Pro
   305                 310                 315
<210> 5
   <211> 906
   <212> DNA
   <213> Homo sapiens
<220>
   <221> CDS
   <222> (1)..(906)
   <223> CD34 (deleted variant)
<400> 5
   atg ccg cgg ggc tgg acc gcg ctt tgc ttg ctg agt ttg ctg cct tct      48
   Met Pro Arg Gly Trp Thr Ala Leu Cys Leu Leu Ser Leu Leu Pro Ser
   1               5                   10                  15
   ggg ttc atg agt ctt gac aac aac ggt act gct acc cca gag tta cct      96
   Gly Phe Met Ser Leu Asp Asn Asn Gly Thr Ala Thr Pro Glu Leu Pro
               20                  25                  30
   acc cag gga aca ttt tca aat gtt tct aca aat gta tcc tac caa gaa      144
   Thr Gln Gly Thr Phe Ser Asn Val Ser Thr Asn Val Ser Tyr Gln Glu
           35                  40                  45
   act aca aca cct agt acc ctt gga agt acc agc ctg cac cct gtg tct      192
   Thr Thr Thr Pro Ser Thr Leu Gly Ser Thr Ser Leu His Pro Val Ser
       50                  55                  60
   caa cat ggc aat gag gcc aca aca aac atc aca gaa acg aca gtc aaa      240
   Gln His Gly Asn Glu Ala Thr Thr Asn Ile Thr Glu Thr Thr Val Lys
   65                  70                  75                  80
   ttc aca tct acc tct gtg ata acc tca gtt tat gga aac aca aac tct      288
   Phe Thr Ser Thr Ser Val Ile Thr Ser Val Tyr Gly Asn Thr Asn Ser
                   85                  90                  95
   tct gtc cag tca cag acc tct gta atc agc aca gtg ttc acc acc cca    336
   Ser Val Gln Ser Gln Thr Ser Val Ile Ser Thr Val Phe Thr Thr Pro
               100                 105                 110
   gcc aac gtt tca act cca gag aca acc ttg aag cct agc ctg tca cct    384
   Ala Asn Val Ser Thr Pro Glu Thr Thr Leu Lys Pro Ser Leu Ser Pro
           115                 120                 125
   gga aat gtt tca gac ctt tca acc act agc act agc ctt gca aca tct    432
   Gly Asn Val Ser Asp Leu Ser Thr Thr Ser Thr Ser Leu Ala Thr Ser
       130                 135                 140
   ccc act aaa ccc tat aca tca tct tct cct atc cta agt gac atc aag    480
   Pro Thr Lys Pro Tyr Thr Ser Ser Ser Pro Ile Leu Ser Asp Ile Lys
   145                 150                 155                 160
   gca gaa atc aaa tgt tca ggc atc aga gaa gtg aaa ttg act cag ggc    528
   Ala Glu Ile Lys Cys Ser Gly Ile Arg Glu Val Lys Leu Thr Gln Gly
                   165                 170                 175
   atc tgc ctg gag caa aat aag acc tcc agc tgt gcg gag ttt aag aag    576
   Ile Cys Leu Glu Gln Asn Lys Thr Ser Ser Cys Ala Glu Phe Lys Lys
               180                 185                 190
   gac agg gga gag ggc ctg gcc cga gtg ctg tgt ggg gag gag cag gct    624
   Asp Arg Gly Glu Gly Leu Ala Arg Val Leu Cys Gly Glu Glu Gln Ala
           195                 200                 205
   gat gct gat gct ggg gcc cag gta tgc tcc ctg ctc ctt gcc cag tct    672
   Asp Ala Asp Ala Gly Ala Gln Val Cys Ser Leu Leu Leu Ala Gln Ser
       210                 215                 220
   gag gtg agg cct cag tgt cta ctg ctg gtc ttg gcc aac aga aca gaa    720
   Glu Val Arg Pro Gln Cys Leu Leu Leu Val Leu Ala Asn Arg Thr Glu
   225                 230                 235                 240
   att tcc agc aaa ctc caa ctt atg aaa aag cac caa tct gac ctg aaa    768
   Ile Ser Ser Lys Leu Gln Leu Met Lys Lys His Gln Ser Asp Leu Lys
                   245                 250                 255
   aag ctg ggg atc cta gat ttc act gag caa gat gtt gca agc cac cag    816
   Lys Leu Gly Ile Leu Asp Phe Thr Glu Gln Asp Val Ala Ser His Gln
               260                 265                 270
   agc tat tcc caa aag acc ctg att gca ctg gtc acc tcg gga gcc ctg    864
   Ser Tyr Ser Gln Lys Thr Leu Ile Ala Leu Val Thr Ser Gly Ala Leu
           275                 280                 285
   ctg gct gtc ttg ggc atc act ggc tat ttc ctg atg aat tga            906
   Leu Ala Val Leu Gly Ile Thr Gly Tyr Phe Leu Met Asn
       290                 295                 300
<210> 6
   <211> 301
   <212> PRT
   <213> Homo sapiens
<400> 6
   Met Pro Arg Gly Trp Thr Ala Leu Cys Leu Leu Ser Leu Leu Pro Ser
   1               5                   10                  15
   Gly Phe Met Ser Leu Asp Asn Asn Gly Thr Ala Thr Pro Glu Leu Pro
               20                  25                  30
   Thr Gln Gly Thr Phe Ser Asn Val Ser Thr Asn Val Ser Tyr Gln Glu
           35                  40                  45
   Thr Thr Thr Pro Ser Thr Leu Gly Ser Thr Ser Leu His Pro Val Ser
       50                  55                  60
   Gln His Gly Asn Glu Ala Thr Thr Asn Ile Thr Glu Thr Thr Val Lys
   65                  70                  75                  80
   Phe Thr Ser Thr Ser Val Ile Thr Ser Val Tyr Gly Asn Thr Asn Ser
                   85                  90                  95
   Ser Val Gln Ser Gln Thr Ser Val Ile Ser Thr Val Phe Thr Thr Pro
               100                 105                 110
   Ala Asn Val Ser Thr Pro Glu Thr Thr Leu Lys Pro Ser Leu Ser Pro
           115                 120                 125
   Gly Asn Val Ser Asp Leu Ser Thr Thr Ser Thr Ser Leu Ala Thr Ser
       130                 135                 140
   Pro Thr Lys Pro Tyr Thr Ser Ser Ser Pro Ile Leu Ser Asp Ile Lys
   145                 150                 155                 160
   Ala Glu Ile Lys Cys Ser Gly Ile Arg Glu Val Lys Leu Thr Gln Gly
                   165                 170                 175
   Ile Cys Leu Glu Gln Asn Lys Thr Ser Ser Cys Ala Glu Phe Lys Lys
               180                 185                 190
   Asp Arg Gly Glu Gly Leu Ala Arg Val Leu Cys Gly Glu Glu Gln Ala
           195                 200                 205
   Asp Ala Asp Ala Gly Ala Gln Val Cys Ser Leu Leu Leu Ala Gln Ser
       210                 215                 220
   Glu Val Arg Pro Gln Cys Leu Leu Leu Val Leu Ala Asn Arg Thr Glu
   225                 230                 235                 240
   Ile Ser Ser Lys Leu Gln Leu Met Lys Lys His Gln Ser Asp Leu Lys
                   245                 250                 255
   Lys Leu Gly Ile Leu Asp Phe Thr Glu Gln Asp Val Ala Ser His Gln
               260                 265                 270
   Ser Tyr Ser Gln Lys Thr Leu Ile Ala Leu Val Thr Ser Gly Ala Leu
           275                 280                 285
   Leu Ala Val Leu Gly Ile Thr Gly Tyr Phe Leu Met Asn
       290                 295                 300
<210> 7
   <211> 36
   <212> DNA
   <213> Artificial sequence
<220>
   <223> Description of the artificial sequence: Primer CD34fw
<400> 7
   aaggaaaaaa gcggccgcca tgccgcgggg ctggac 36
<210> 8
   <211> 31
   <212> DNA
   <213> Artificial sequence
<220>
   <223> Description of the artificial sequence: Primer CD34rev
<400> 8
   taagcttatc acaattcggt atcagccacc a 31
<210> 9
   <211> 48
   <212> DNA
   <213> Artificial sequence
<220>
   <223> Description of the artificial sequence: Primer CD341rev
<400> 9
   caataagctt atcatggttc tagttccagc ctttctcct gtggggct 48
<210> 10
   <211> 34
   <212> DNA
   <213> Artificial sequence
<220>
   <223> Description of the artificial sequence: Primer CD34srev
<400> 10
   caataagctt atcaattcat caggaaatag ccag 34

## Description

The present invention concerns the use of CD34 or a polypeptide derived therefrom as cell-surface/gene transfer marker. In particular the object of the invention is a gene transfer vector and host cells transduced by the vector, the vector containing a transgene and a nucleic acid sequence coding for CD34, a fragment of the same, or a variant of the same. The vector is particularly suitable for use in procedures for identification and/or selection of genetically modified cells and in gene therapy. The invention thus further concerns kits for carrying out these procedures and the use of the vector *in vitro* and *in vivo.*

Marker genes are important aids, enabling the identification and selection of genetically modified cells in experimental immunology, haematology and gene therapy. The low affinity nerve growth factor receptor (complete length, LNGFR, or intracytoplasmatically truncated, ΔLNGFR), variants of human and murine surface antigens such as CD24, CD2, CD4 and the enhanced green fluorescent protein (EGFP) are currently most frequently used for this purpose. None of these markers however appears to be optimal for clinical practice. A marker suitable for this purpose should be of human origin, to avoid an immune response. Moreover it should only be presented on the genetically modified cells, without being released in the extracellular space. In particular, the markers which can be used for clinical practice should not disturb the physiological functions of the target cells. The surface markers used hitherto are only conditionally suitable, especially for clinical use, i.e. within the framework of gene therapy (cf. B. Fehse et al., Gene Therapy 5 (1998) 429-430).

The task of the present invention is therefore to make available surface markers for the identification and selection of genetically modified cells, which do not have the disadvantages observed in connection with the markers used in the state of the art. The markers should in particular be suitable, within the framework of a gene therapeutic protocol, for the selection/identification of transduced cells of the haematopoietic system, especially primary human or murine T-lymphocytes, without interfering with the haematopoiesis.

According to the invention the task is solved by use of the CD34 surface antigen, a fragment of the same or a variant of the same, wherein said fragment or variant is a truncated form of the CD34 surface antigen where protein kinase C (PKC) phosphorylation sites are deleted, or is a cytoplasmatically completely or partially deleted variant of the CD34 surface antigen. In particular the task is solved by the object of the attached claims.

Within the framework of the present invention it was surprisingly ascertained that CD34 is excellently suited for the identification and/or selection of genetically modified cells, with it also being possible to use primary T-lymphocytes as target cells, which after transduction with a gene transfer vector within the framework of agene therapeutic protocol, can be delivered to a receiver organism, without this resulting in disturbances of the haematopoiesis. Although haematopoietic stem cells express CD34, the haematopoiesis is, unexpectedly, not negatively influenced by the surface marker expressed by the genetically modified cells. In particular it was unexpectedly ascertained that the expression of CD34 on the target cells does not result in a negative influence on cell function and/or cell differentiation. It was moreover possible to show that the expressed surface marker is not toxic to the target cells and - as this is a question of a human protein - it does not provoke an immunogenic effect in the recipient.

As CD34 is naturally expressed only on very few cell types, such as human haematopoietic progenitor or stem cells, CD34 is suitable, in a particularly advantageous way, for the purification, enrichment and analysis of cells which do not naturally express CD34, but especially for the identification and/or selection of genetically modified (transduced) cells, for which technologies exist in the state of the art, and are in particular also permitted for clinical practice, including well characterised monoclonal antibodies, which permit enrichment of the marked cells with a high degree of purity according to GMP (good manufacturing practice) conditions.

For the enrichment and analysis and/or detection of cells which do not naturally express CD34, a nucleic acid sequence coding for CD34 (or for a fragment of the same or a variant of the same) can by means of a vector be introduced into these cells in a form suitable for expression there.

For the marking of genetically modified cells, according to the invention a nucleic acid sequence coding for CD34 (or for a fragment of the same or a variant of the same, wherein said fragment or variant is a truncated form of the CD34 surface antigen where protein kinase C (PKC) phosphorylation sites are deleted, or is a cytoplasmatically completely or partially deleted variant of the CD34 surface antigen) is transferred into the target cell together with the gene sequence (transgene) used for the actual transduction. In this case the term "transgene" is taken to mean a nucleic acid sequence which codes for a protein, polypeptide or peptide, which is expressed in the target cell (or host cell) and confers a new property or function upon this cell. The transgene thus differs from others within the framework of the transduction of transferred nucleic acid sequences in that, the expression product formed in the host cell directly influences the physiological properties and/or the functionality of the cell. With regard to a gene therapeutic application the transgene is a nucleic acid sequence coding for a therapeutically effective protein, polypeptide or peptide.

The object of the present invention is therefore a (gene transfer) vector, which contains
(a) a transgene (optional) and
(b) a nucleic acid sequence coding for a surface marker,
the surface marker being the CD34 surface antigen or a fragment of the same, wherein said fragment or variant is a truncated form of the CD34 surface antigen where protein kinase C (PKC) phosphorylation sites are deleted, or is a cytoplasmatically completely or partially deleted variant of the CD34 surface antigen. Also included according to the invention are all conceivable variants due to amino acid exchanges.

According to a preferred embodiment of the invention, the CD34 surface antigen has the sequence indicated in SEQ ID NO:2, wherein the nucleic acid sequence coding for this protein is preferably the sequence indicated in SEQ ID NO:1. Due to the degeneration of the genetic code, according to the invention variants and mutants of this nucleic acid sequence are included, which code for the same protein. The invention further relates to fragments, mutants and variants of the sequence indicated in SEQ ID NO:1, which code for a protein, polypeptide or peptide comparable with CD34, which has the same or essentially identical properties and is suitable as a surface marker for the identification and/or selection of genetically modified cells.

Within the framework of the present invention, it has been shown that nucleic acid sequences are advantageous, which code for a truncated form of the CD34 surface antigen, i.e. for variants in which protein kinase C (PKC) phosphorylation sites are deleted. Also included according to the invention are thus cytoplasmically completely or partially deleted variants of the CD34 surface antigen and gene transfer vectors which contain nucleic acid sequences coding for these variants. This nucleic acid sequence is preferably the sequence indicated in SEQ ID NO:3 or 5.

Within the framework of the present invention, it has been shown that the expression of the truncated/deleted variants of the CD34 protein according to SEQ ID NO:4 and/or 6 allows genetically transduced cells to be detected and selected in an especially suitable way. As the two polypeptides differ from each other only in that the truncated variant (tCD34) is 15 amino acids longer than the deleted variant (dCD34), other variants can naturally also be taken into consideration, whose length lies between the truncated and the deleted variant. Correspondingly the nucleic acid sequence coding for a surface marker will have a length which lies between the lengths of the sequences indicated in SEQ ID NO:3 and 5.

Within the framework of the present invention it has been ascertained that the truncated variant tCD34, compared with the deleted variant, dCD34, has the advantage that the surface antigen is anchored more stably in the membrane of the transduced cells, whereby the identification and selection of the genetically modified cells are clearly improved due to the lower release in the extracellular space. According to a particular embodiment of the invention the nucleic acid sequence coding for the surface marker has in particular the sequence indicated in SEQ ID NO:3 or a sequence derived therefrom by mutation. Due to the degeneration of the genetic code other nucleic acid sequences coding for tCD34 according to SEQ ID N0:4 also naturally come under consideration. Also, nucleic acid sequences which code for variants of the truncated CD34 surface antigen are included (including those obtained by amino acid exchanges), which have the same or essentially identical properties to the polypeptide indicated in SEQ ID NO:4.

The vector according to the invention can be a non-viral, viral or retroviral vector. A retroviral vector obtained according to a preferred embodiment of the invention, which codes for tCD34, was deposited on 27.03.2000 at the Deutsche Sammlung fur Mikroorganismen und Zellkulturen GmbH (DSMZ), Mascheroder Weg 1b, D-38124 Brunswick (Braunschweig), Germany, under accession No. DSM 13396.

According to a preferred embodiment of the invention the gene transfer vector further contains a nucleic acid sequence coding for a further surface marker such as CD2, EGFP etc., or especially for a therapeutic gene (such as adenosindesaminase (ADA) for healing of the serious immuno-deficiency syndrome ADA-SCID; or others) or a suicide gene. A suicide gene as transgene enables later elimination of transduced cells.

The term "transgene" according to the invention is taken to mean any nucleic acid sequence (foreign gene) which is not naturally contained in the genome of the host or receiver organism or in the vector genome and/or any nucleic acid sequence which is to be transferred to a receiver/host (receiver or host cell).

A further object of the invention is a host cell which is transduced with an aforementioned vector. This host cell is distinct in that in addition to the transgene it also contains the nucleic acid sequence coding for the surface marker and the marker is expressed on the surface of the host cell. The host cell can be a human or non-human (e.g. murine) cell, with (human) T-lymphocytes being preferred.

The invention includes the use of a nucleic acid sequence (marker gene) coding for the CD34 surface antigen or a fragment of the same or a mutant, variant of the same (marker) for the detection of genetically modified cells, in which the nucleic acid sequence is inserted into a gene transfer vector used for genetic modification, which contains a nucleic acid sequence (transgene) to be transferred into the cells, the marker gene and the vector being chosen so that the marker is expressed on the surface of the cells transduced with the vector, the genetically modified cells being identified by specific detection of the marker. The object of the present invention is thus a method to detect genetically modified cells, in which the cells are transduced with a "vector" and the transduced cells are detected by means of selective detection of the marker expressed on the surface of the cells. This detection can be carried out using various methods known to the expert, such as by means of flow cytometric analysis (cf. Fehse et al., Hum. Gene Ther. 8 (1997) 1815-1824) or immunohistochemical methods (Ruggieri et al., Human Gene Ther. 8 (1997) 1611-1623) using monoclonal antibodies.

A further object of the invention is a method for selection of genetically modified cells, in which the cells are transduced with an aforementioned vector and the transduced cells are bound to an agent specific to the surface marker, especially a (monoclonal) antibody, and the cells are thus separated from the genetically non-modified cells (e.g. by magnetic cell sorting, Fehse et al., Hum. Gene Ther. 8 (1997) 1815-1824; or other immuno-adhesion techniques or fluorescence-activated cell sorting, FACS. cf. e.g. Phillips et al., Nat. Med. 2 (1996) 1154-1156).

As already mentioned, the cells used in the method according to the invention are human cells, preferably human T-lymphocytes.

A further object of the invention is a kit to carry out the aforementioned detection method, containing an aforementioned vector, means for the specific detection of the surface marker (including means for carrying out flow cytometry or immunohistochemistry), especially monoclonal antibodies as well as further agents and adjuvants needed to carry out the detection, such as suitable buffers and blocking solutions.

A further object of the inventions is a kit to carry out the aforementioned selection method, containing an aforementioned vector, means for specific binding of the surface marker, such as antibodies coupled to magnetic and/or paramagnetic beads, and further agents and adjuvants necessary to carry out the selection.

As already mentioned above, the vector according to the invention is especially distinguished by its suitability for gene therapeutic applications. The invention therefore further relates to the application of the aforementioned vector for the production of a gene therapeutic drug, especially for the transduction of (human) T-lymphocytes, and a gene therapeutic drug containing this vector. Also included is the use of (human) T-lymphocytes which are transduced with an aforementioned vector, for gene therapeutic treatment. Finally the- invention concerns a gene therapeutic drug containing (human) T-lymphocytes which are transduced with the vector according to the invention.

The present invention is described in more detail below with reference to examples, figures and a sequence protocol.

### Examples

In the following examples the following general techniques are used:
a) Cultivation of primary cells and cell lines.
   Mononuclear cells were isolated from the blood of healthy donors by Ficoll gradient centrifugation (Biochrom, Berlin, Germany) (900 g, 20 min.). T-cells were stimulated with 10 ng/ml OKT-3 (Cilag, Neuss, Deutschland) at a density of 2x10¹⁶/ml in the presence of 100 U/ml IL-2 (Roche, Mannheim, Germany) in X-Vive 10 (BioWhittaker, Verviers, Belgium), which contained 8% autologous serum (F.A. Ayuk et al., Gene Ther. 6 (1999) 1788-1792). Jurkat and K562 cells were kept in RPMI 1640 which contained 10% foetal calf serum (FCS) and 2 mM glutamine (all from Gibco BRL, Karlsruhe, Germany). The producer cells of retroviral vectors Phoenix ampho (http://www.standford.edu/group-/holan/NL-phnxr.html, Grignani, F., Kinsella, T., Mencarelli, A. et al., Cancer Res. 58 (1998) 14-19) and PG13 (ATCC CRL-10686, http://www.ATCC.-org- cf. A.D. Miller et al., J. Virol. 65 (1991) 2220-2224) were kept in Dulbecco's modified Eagle-Medium (DMEM + Glutamax; Gibco BRL), supplemented with 10% heat-inactivated FCS and sodium pyruvate (final concentration 1mM, Gibco BRL). All cells were kept at 37°C in a humidified atmosphere in CO₂ incubators (Heraeus, Hannover, Germany).
b) Gene transfer in K562, Jurkat and primary human T-cells.
   Primary T-cells were stimulated with OKT-3 (see above) and cultivated for 3 days in the presence of 100 U/ml IL-2. Jurkat and K562 cells were transduced without prior stimulation. 3x10⁶ cells were suspended in 3 ml filtered Retrovirus-containing supernatant, and 4 µg/ml protamine sulphate (Merck, Darmstadt, Germany) were added. The cells were centrifuged for one hour at 2000 U/min in 6-well-TC plates (Becton Dickinson). Transductions were repeated after 24 hours. The cells were kept in culture for at least 2 days before determination of the gene transfer efficiency.
c) Southern, Northern and Western blot.
   Southern, Northern and Western blots were carried out in accordance with standard protocols (F.M. Ausubel et al., Short protocols in molecular biology, 2nd edition, John Wiley and Sons, New York, 1992). Southern and Northern blots were hybridised with radioactively (³²P) marked f1CD34. For Western blots and immunoprecipitations cell lysates or cell culture supernatants were used as indicated below.

### Example 1:

### Cloning, production and genetic characterisation of flCD34-, tCl·34-and dCD34- expressed retroviral vectors

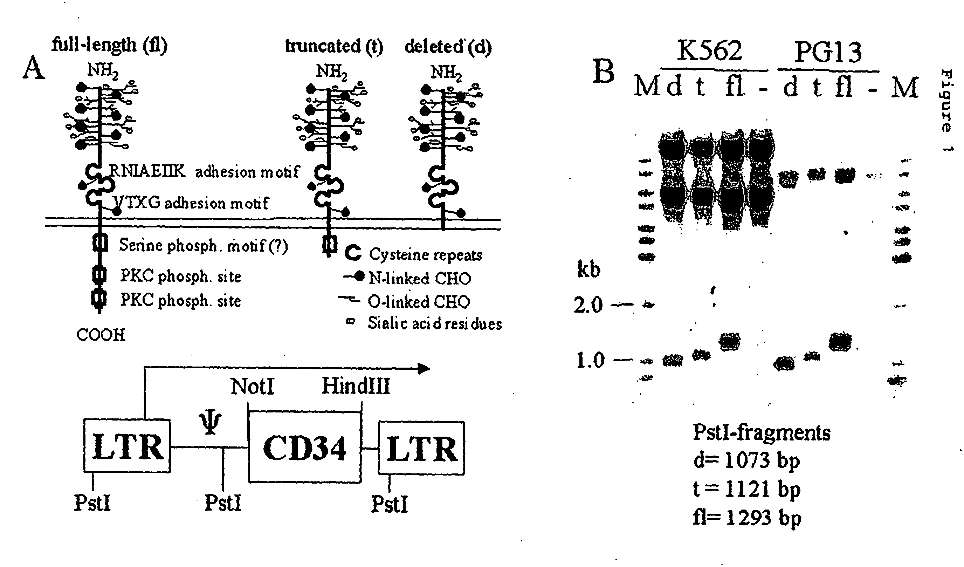

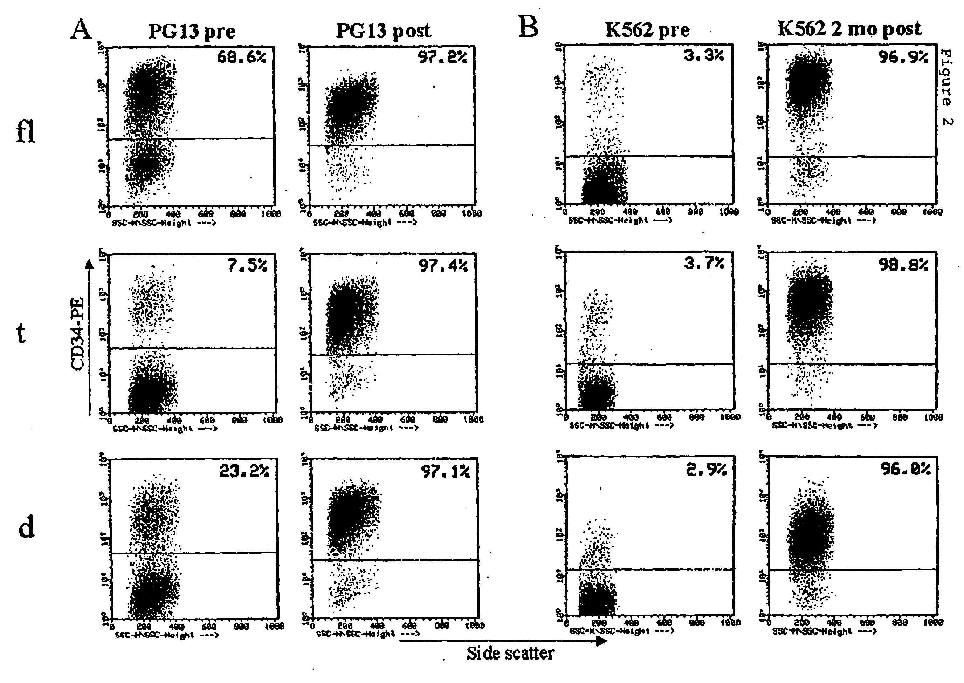

The three types of CD34 which were analysed in this study are represented in Figure 1a. The cDNAs for flCD34, tCD34 and dCD34 were obtained by means of a RT-PCR with RNA, which had been obtained from human TF1 leukaemia cells, which express CD34 endogenously (Figure 2b and data from flow cytometry, not shown). In this procedure the RNA from human CD34⁺-leukaemia cells (TF1) was isolated using the RNeasy Mini Kits (Qiagen, Hilden, German). cDNA was synthesised using an oligo-dT primer and Superscript^{™} Reverse Transkriptase (Gibco BRL) according to the manufacturer's instructions. The open reading frame for flCD34 was obtained by means of PCR using Pfu polymerase (Stratagene, Amsterdam, Netherlands) and the primers CD34fw 5' -AAGGAAAAAAGCGGCCGCCATGCCGCGGGGCTGGAC-3' (SEQ ID NO: 7) and CD34rev 5' -TAAGCTTATCACAATTCGGTATCAGCCACCA-3' (SEQ ID NO: 8). The flCD34 cDNA served as matrix for the production of tCD34 and dCD34 using the primers CD34fw and CD341rev (5'-CAATAAGCTTATCATGGTTCTAGTTCCAGCCTTTCTCCTGTGGGGCT-3'; SEQ ID NO: 9) or CD34fw + CD34srev (5' -CAATAAGCTTATCAATTCATCAGGAAATAGCCAG-3' ; SEQ ID NO: 10).

The sequencing of the subcloned PCR products revealed an A-G exchange (by comparison with the sequences M81104 and S53811 in GenBank; cf. D.L. Simmons et al., J. Immunol. 148 (1992) 267-271), which leads to an exchange of glutamate for lysin in codon 349 of flCD34. This codon lies in the cytoplasmatic section of the protein and is not present in tCD34 and dCD34, so it was not modified for this study. All variants have the shorter signal peptide which is described in GenBank M81104 (cf. D.S. Simmons et al., J. Immunol. 148 (1992) 267-271). The cDNAs were cloned into the polylinker region of pKS and from the retroviral expression vector pSFα11 using NotI and HindIII restriction sections. The retroviral vectors (Fig. 1A) use the enhancer/promoter of a variant of the murine spleen focus-forming virus (SFFVp) for initiation of the transcription, which shows moderate activity in murine and human T-cells (C. Baum et al., Virol. 88 (1995) 7541-7547; own results). They also contain an untranslated gag-replacement (GR) leader region which chiefly prevents the expression of aberrant proteins or peptides (M. Hildinger et al., J. Virol. 73 (1999) 4083-4089). Retroviral vectors with high infectious titres were obtained after transduction of the resultant plasmids in Phoenix-ampho packing cells. In this procedure Phoenix cells (amphotropic env-protein) were transduced using the calcium phosphate transduction kit (PeqLab, Erlangen, Germany). In some experiments a plasmid expression vector was co-transduced, which codes for the glycoprotein of the vesicular stomatitis virus (VSV-G), to obtain mixed amphotrope/VSV-G pseudotypes. This led to mixed pseudotypes with titres greater than 10⁶/ml, which allowed the infection of various human and murine cell types. To obtain stable retroviral producer cells and to test the integrity and capacity of the retroviral constructs, retroviral packing cells PG13 and human K562 erythroleukaemia cells were transduced with the supernatants of the transduced Phoenix cells. By subsequent sorting of CD34⁺-cells and use of MACS technology (Magnetic cell sorting ; method for enrichment of microbeads-antibody-marked cells by means of special MACS columns which are placed in a strong magnetic field) PG13-producer cell (Gibbon-Ape Leukaemia virus env-proteins) mass cultures could be established. PG13 clones were isolated following limited dilution. Southern-blot analysis of transduced polyclonal PG13 and K562-cells showed the genetic stability of the constructs (Figure 1b), whereby it was ensured that the following analyses were carried out with cells, in which the transgenes were correctly processed. Supernatants of the cells were collected after six hours' incubation at 37'C in X-vivo 10 (F.A. Ayuk et al., Gene Ther. 6 (1999) 1788-1792). Viral titres were determined by transduction of Jurkat cells with serial dilutions of virus-containing supernatants and subsequent FACS analysis (B. Fehse et al., Hum. Gene Ther. 8 (1997) 1815-1824).

### Example 2

### Enrichment of genetically modified cells by CD34

The flow cytometry showed that in murine fibroblast PG13 cells and in human K562 haematopoietic cells, the retroviral vectors expressed all three CD34 variants (flCD34, tCD34 and dCD34) each in large quantities. The differences in transfer-efficiencies correspond to the titres of the packaging cells. Polyclonal populations of FG13 and K562 cells, which expressed the three different versions of CD34 could easily be enriched to a high purity using cell sorting based on immuno-affinity (MACS technology) (Figure 2A, B). To do so, the cells were enriched three days after transduction using the CD34 progenitor cell isolation kit (Miltenyi Biotec, Bergisch-Gladbach, Germany) in accordance with the manufacturer's instructions (B. Fehse et al., Hum. Gene Ther. 8 (1997) 1815-1824). After enrichment the cells were marked with phycoerythrin-coupled anti-CD34 (HPCA-2), not interfering with the antibodies used for enrichment. The new phenotype remained stable in culture for several months both for K562 (Fig. 2B) and also for PG13 (data not shown) . No influence of the variants on the proliferation of the transduced cells could be detected. However flow cytometry showed that the cell surface expression of dC34 was weaker than that of the other two variants.

### Example 3:

### The residual cytoplasmatic part of tCD34 is involved in the membrane anchoring of the cell surface molecule.

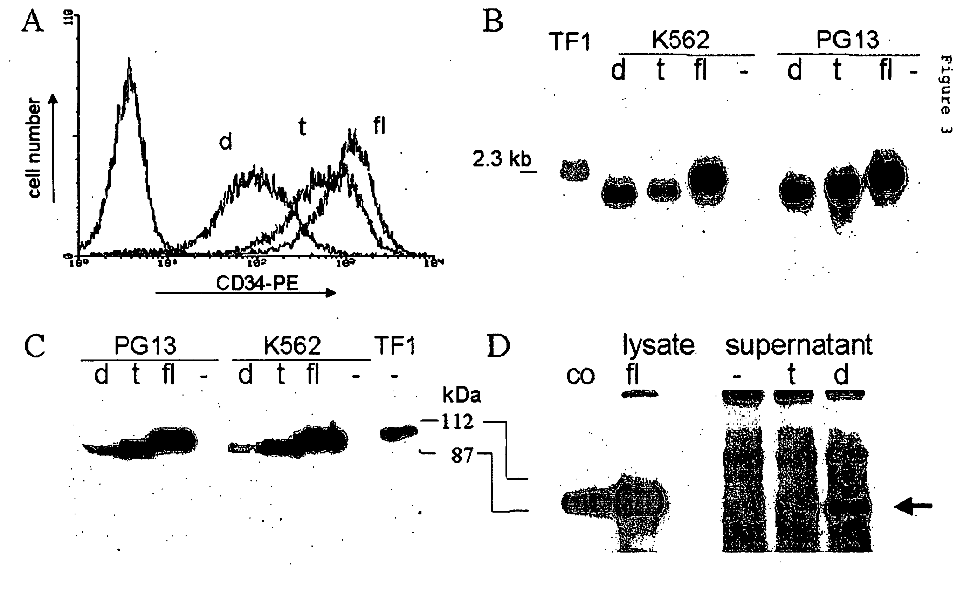

To examine the mechanisms on which the observed expression differences are based, the three transduced variants of CD34 were examined at the level of the transcript and of the protein. This took place using polyclonal populations of K562 and PG13 cells, which had been transduced with the three versions of the retroviral CD34 vectors and which had been immuno-selected for expression of CD34. The histogram of cell surface marking with CD34, with which the different variants were compared, demonstrated that the expression of dCD34 was an order of magnitude lower than that of tCD34, which was just as strongly represented on the cell surface as flCD34 (Figure 3A). Comparable data could be achieved with human Jurkat lymphocytes (not shown). Northern blot analysis (Figure 3B) confirmed that the three variants had different transcript lengths and identical total expression rates. The stronger signal from flCD34 in K562 cells could be explained by a higher loading with RNA, which was revealed by methylene-blue staining of the membrane before hybridisation (not shown). This result shows that the 3' end of the CD34 cDNA contains no sequences that influence the processing of the RNA. The loss of expression must therefore originate from differences in the processing of the CD34 protein (Figure 3C, D).

Western blot analyses of the cell lysates confirmed the results obtained by flow cytometry. Figure 3C shows the weaker expression of dCD34, compared with the two naturally occurring forms tCD34 and flCD34. This argues against the unlikely possibility that dCD34 is retained in the cytoplasma. The Western blot showed that dCD34, like tCD34, has a reduced molecular weight compared with flCD34.

In summary these results show that the membrane anchoring of dCD34, which lacks the cytoplasmatic part, but still contains the complete transmembrane-domain, can be unstable. To investigate this question, non-concentrated cell culture supernatants of K562 cells which had been transduced either with dCD34 or tCD34 (Figure 3D) were immuno-precipitated. In fact CD34 was detected in clearly increased quantities in the supernatants of the dCD34 expressing cells. Therefore the reduced cell surface expression of dCD34 can be explained by release from the membrane. From this it can be concluded that the residual cytoplasmatic part of the tCD34 has an important function in membrane anchoring and through this prevents the release of the cell surface protein.

### Example 4

### Expression of tCD34 in human T-lymphocytes

On the basis of the results available, tCD34 was selected as the most interesting variant for the marking of cell surfaces and the immuno-selection of genetically modified cells. An essential application of this technology is the enrichment of genetically modified lymphocytes for use in adoptive immunotherapy in patients (C. Bonini et al., Science, 276 (1997) 1719-1724; P. Tiberghien et al., Hum. Gene Ther. 8 (1997) 615-624. It was therefore examined, whether the retroviral vector-mediated expression of tCD34 in human T-cells is feasible.

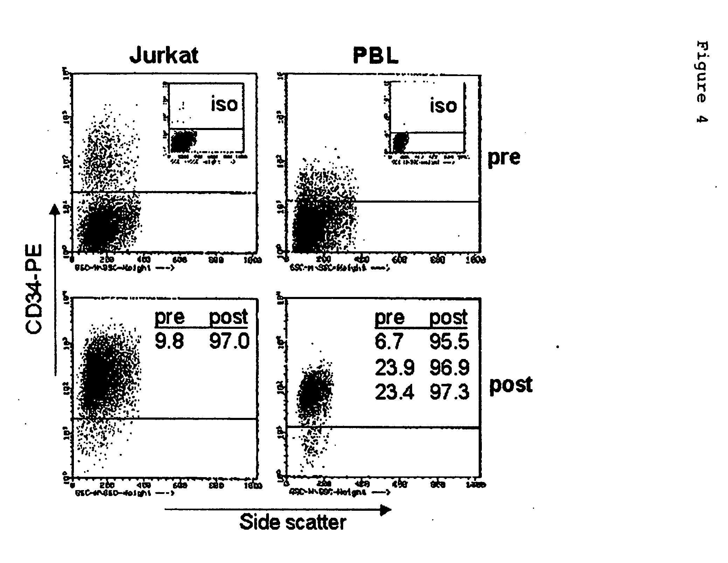

The transduction of human T-cells is best carried out using retroviral vectors which are pseudo-standardized with the env-protein of the Gibbon Ape Leukaemia Virus (GALV). These vectors can be produced in PG13 cells (F.A. Ayuk et al., Gene Ther. 6 (1999) 1788-1792; B.A. Bunnell et al., Blood 89 (1997) 1987-1995). Stable clones of PG13 cells, which express the retroviral vector Sfα11tCD34 at high titres were obtained by limited dilution of the corresponding mass culture. On 27.03.2000 a specimen of this vector was deposited with the DSMZ in Brunswick (see above) under no. DSM 13396. Supernatants of these producer cells were used for the gene transfer in human Jurkat T-lymphoblastoma cells (Figure 4A) and in primary peripheral blood lymphocytes (PBLs) which had been stimulated with IL-2 and OKT-3 (Figure 4B) . As was determined by flow cytometry, the expression of tCD34 in Jurkat cells was slightly higher than in primary T-cells. A similar difference was also observed with a retroviral vector which contains identical transcription control elements, but expresses EGFP instead of tCD34 (not shown). The expression of tCD34 was strong enough to enable a separation of these cells using MACS technology both with Jurkat and PBLs. The immuno-selected cells were always obtained with high purity (Figure 4). These cells were observed in culture for up to a week. They remained CD34 positive and showed no obvious changes in proliferation or morphology.

### Example 5:

### The use of tCD34 to track genetically modified murine erythroid, myeloid and lymphoid cells in vivo.

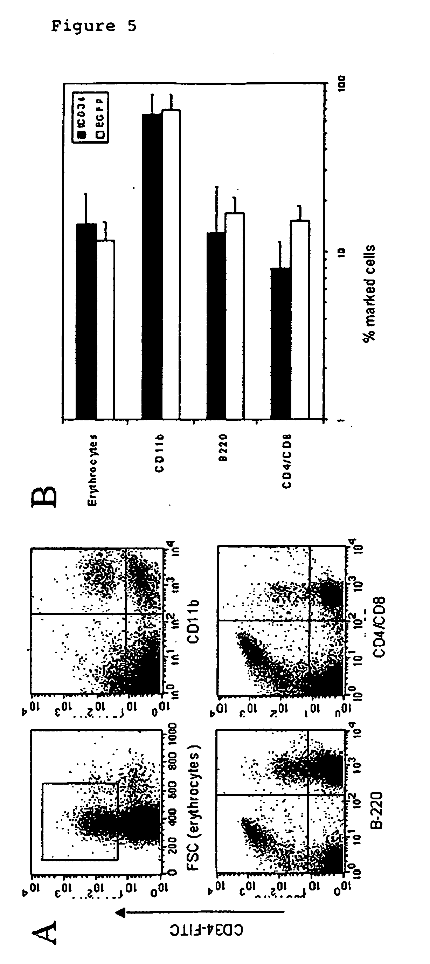

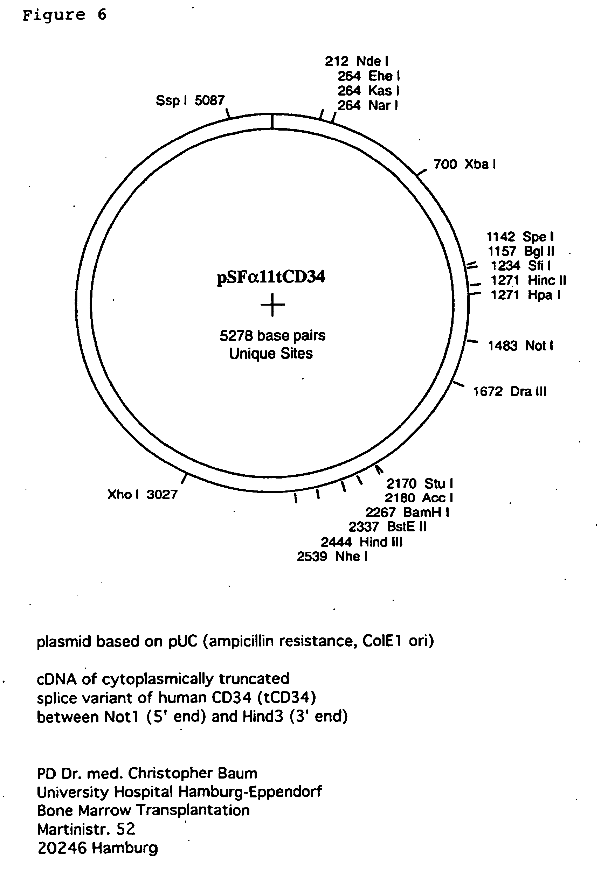

Finally it was examined whether tCD34 can be used to mark retrovirally modified murine haematopoietic cells, including primary T-cells in vivo, which are obtained after transplantation with multi-potent precursor cells. Non-fractionated mononuclear bone marrow cells were transduced with retroviral vectors which express tCD34 or as a control EGFP. These vectors were identical with regard to the cis-acting elements, which control the gene expression (Figure 1). In particular the cells were obtained as follows: bone marrow cells were obtained from the fibia and the femurs of male C57B1/6J donor mice (age 12-16 weeks) 4 days after intraperitoneal delivery of 5-fluorouracil (Sigma) (150 mg/kg). Mononuclear cells were prestimulated in IMDM medium (Iscove's Modified Dulbecco's Medium), which had been supplemented with 20% fetal calf serum, 2mM glutamate, 100 U/ml penicillin, 100 µg/ml streptomycin and a normal growth factor cocktail containing murine IL-3 (10 ng/ml), human IL-6 (200 U/ml) and murine SCF (50 ng/ml). Recombinant growth factors were obtained from Strathmann Biotech (Hannover, Germany). After two days' prestimulation cell-free supernatants of mixed ampho/VSV-pseudo-standardized retroviral particles were added. The multiplicity of infection (MOI) amounted to 0.7 infectious particles per cell, calculated from previous titration of aliquots of supernatants on SC-1 fibroblasts. Polybrene (sigma) (4 µg/ml) was added and the cells were centrifuged for an hour at 2000 U/min. This process was repeated three times within 48 hours, with a pause of at least 8 hours between the individual transduction steps. Comparable transduction rates were achieved, by using cell-free supernatants with equivalent titres. One day after the transduction was completed, the cells were transplanted into the tail veins of lethally irradiated (10 Gy) female recipients (n=6 for each vector) at a dose of 2.2×10⁶ cells per mouse. Nine weeks after the transplantation, the peripheral blood cells were analysed by means of flow cytometry with respect to the expression of the transgene in erythroidal cells (determined by scatter properties), and in myeloid cells, B-lymphocytes and T-lymphocytes (identified using the monoclonal antibodies CD11b, B220, and a combination of CD4 and CD8). Cells of these lines, including T-lymphocytes, expressed tCD34 in easily detectable quantities (Figure 5A), from which it can be concluded that the transgene and surface-marked cells normally differentiate in vivo, and transgene expression remains stable. Whilst EGFP and tCD34 could be found in a comparable frequency in myeloid and erythroid cells, it was possible to observe a tendency for lymphoid cells with tCD34 to become somewhat less marked (Figure 5B).

### Legends to figures

### Figure 1

Retroviral vectors for the expression of all three variants of CD34.
(A) (above) Schematic representation of the three CD34 variants, (modified from D.S. Krause et al., Blood 87 (1996) 1-13): The cytoplasmatic tails of flCD34, tCD34 and dCD34 comprise 73, 16 and 1 amino acids; (bottom) proviral form of the retroviral vectors used for expression of CD34. Size of the proviral form is approx. 2.6 kb. The long terminal repeat (LTR) is from a variant of the murine spleen focus forming virus. The untranslated leader region contains the retroviral packing signal (Ψ) without gag sequences. The cDNAs were inserted using NotI and HindIII restriction sites.
(B) Southern blot analysis of the immuno-selected K562 and PG13 cells transduced with the three different retroviral CD34 expression vectors. Genomic DNAs were digested with PstI. Hybridization with the probe human flCD34 revealed correct insert lengths, as indicated. Hybridisation signals of higher molecular weight result from cellular genes. M, DNA molecular weight marker ladder mix (MBI Fermentas, St. Leon-Rot, Germany).

### Figure 2

Enrichment of PG13 and K562 cells based on stable expression of retrovirally transduced CD34.
(A) PG13 cells after transduction with Phoenix supernatants before (PG13 pre, analysed two days after transduction) and immediately after (PG13 post) enrichment using immuno-affinity columns.
(B) K562 cells before (K562 pre, analysed two days after transduction) and two months after (K582 2mo post) enrichment using immuno-affinity columns.

### Figure 3

The cell surface expression of dCD34 is reduced because of release into the cellular supernatant.
(A) Overlay histogram of CD34 expression in uncloned, K562 cells transduced with dCD34 (d), tCD34 (t) or flCD34 (fl); determination by flow cytometry two months after immunoaffinity-based enrichment with a resulting purity of 96%, 98% and 97% respectively; cell-surface expression of dCD34 is about one order of magnitude reduced as compared with tCD34 and flCD34. Similar results were obtained with Jurkat cells (not shown).
(B) Northern blot analysis of total RNA, harvested from mass cultures of K562 and PG13 cells transduced with the three variants of the CD34 expression vectors or untransduced cells (-). TFI cells are shown as a positive control, with an endogenous expression of flCD34 (lower band, approx. 2.3 kb) and tCD34 (upper band, approx. 2.5 kb) (Krause et al., loc. cit., 1996). Comparison with the loading control (methylene-blue stained filter, not shown) confirmed comparable expression levels of all three retroviral RNAs in transduced cells.
(C) Western blot analysis of cell lysates, harvested from transduced and untransduced PG13 and K562 cells (the same cultures as in (A) and (B)). Weaker expression of dCD34 is confirmed. It should be noted that dCD34 and tCD34 have a lower molecular weight (approx. 100 kDa), compared with flCD34 (approx. 110 kDa). 20 µg protein were loaded per lane, CD34 was detected using the monoclonal antibody QBEND 10, HRP conjugated goat-anti-mouse IgG and the SuperSignal^{™} West Pico Chemoluminescence substrate (Pierce, Rockford, Illinois).
(D) Immuno-precipitation with HPCA-2 antibodies from cellular supernatants of K562 cells, which had been transduced with tCD34 (t) or dCD34 (d) or from non-transduced cells (-). The arrow indicates soluble CD34. Cell lysate of K562:flCD34 cells are shown as a positive control (co), just as immuno-precipitated lysates of K562:flCD34 cells. Detection was carried out by means of Western blot, as described above.

### Figure 4

Enrichment of genetically modified human T-cells, including primary peripheral blood lymphocytes (PBL) using retroviral vectors, which expressed tCD34. The isotype control is shown as an insert (iso) in the dot blot analysis, which was obtained from the (pre) magnetic cell sorting (MACS technology). In independent experiments the purity after enrichment (post) was 95.5%, 96.9% and 97.3% for PBL after an initially positive signal of 6.7%, 23.9% and 23.4%

### Figure 5

Expression of tCD34 on murine peripheral blood cells in vivo.
(A) Representative dot blots which show the expression of tCD34 in the blood of mice, status 9 weeks after bone marrow transplantation with retrovirally marked cells. Peripheral blood cells of C57B1/6J mice were obtained by bleeding the tail veins and were tested by means of flow cytometry for expression of tCD34. By means of scatter profile and derivation-specific antibodies, myeloid cells (CD11b), B-cells (B220), T-cells (cocktail of CD4 and CD8) and erythrocytes were differentiated, the latter being determined by size corresponding to the forward scatter (FSC). The markers were adjusted on the basis of isotype controls.
(B) The marking efficiency with tCD34 is comparable with results obtained with EGFP. The multiplicity of the infection was adapted to an equal gene transfer efficiency, as indicated by equal marking in myeloid and erythroid cells. It should be noted that there is a tendency for lymphocytes with tCD34 to be slightly less marked than those with EGFP. The average values are shown (percentage of marker-positive cells) with standard deviations. There were six animals within each experimental group.

### Figure 6

pUC-based plasmid (Ampicillin-resistance, ColEl ori). The cDNA of the cytoplasmatically truncated variant of human CD34 (tCD34) is located between NotI (5'-end) and HindIII (3'-end).

In the plasmid pSFalphalltCD34 the reading frame of tCD34, a splice variant of the human CD34 differentiation antigen, is located between NotI and HindIII. It lies functionally under the control of an eukaryontic promoter with a subsequent 5'-untranslated region (Region 150 bp upstream of XbaI to NotI, comprising sequences of the murine retroviruses MPSV and MESV). The corresponding signal in the long terminal repeat (LTR) of the SFFV retrovirus initiates polyadenylation; this LTR is located between HindIII and XhoI. The transcription-regulating signals are only recognized in the case of transfection in eukaryontic cells. The sequences downstream from XhoI to ca. 150 bp upstream from XbaI comprise the plasmid backbone based in pUC19, which mediates the ampicillin resistance in transformed bacteria and bears the replication origin for the plasmid.

## Claims

1. Gene transfer vector which contains
a) a transgene and
b) a nucleic acid sequence coding for a surface marker,
**characterized in that** the surface marker is the CD34 surface antigen or a fragment of the same or a variant of the same, wherein said fragment or variant is a truncated form of the CD34 surface antigen where protein kinase C (PKC) phosphorylation sites are deleted, or is a cytoplasmatically completely or partially deleted variant of the CD34 surface antigen.

2. Vector according to Claim 1, **characterized in that** the nucleic acid sequence codes for a surface marker in accordance with SEQ ID NO: 2 (CD34), 4 (tCD34) or 6 (dCD34) or for a fragment or a variant of the same, wherein the length of said fragment or variant lies between the length of tCD34 and dCD34.

3. Vector according to Claim 1 or 2, **characterized in that** the nucleic acid sequence encoding the surface marker is the sequence indicated in SEQ ID NO: 1, 3 or 5 or a fragment or variant of the same, wherein the length of said nucleic acid encoding said fragment or variant lies between the lengths of the sequences indicated in SEQ ID NO : 3 and 5.

4. Vector according to Claims 1 to 3, **characterized in that** it is a retroviral vector.

5. Vector according to Claims 1 to 4, **characterized in that** it contains a nucleic acid sequence coding for a further surface marker.

6. Vector with the accession no. DSM 13396.

7. Vector **characterized in that** it contains a nucleic acid sequence coding for the amino acid sequence according to SEQ ID NO: 6.

8. Vector according to Claim 7, **characterized in that** it contains the nucleic acid sequence according to SEQ ID NO: 5.

9. Host cell, **characterized in that** it is transduced with a vector according to Claims 1 to 8.

10. Host cell according to Claim 9, **characterized in that** it is a human cell.

11. Host cell according to Claim 10, **characterized in that** it is a T-lymphocyte.

12. Method for the detection of genetically modified cells, **characterized in that** the cells are transduced with a vector according to Claims 1 to 5 and the transduced cells are identified by detection of the surface marker.

13. Method for the selection of genetically modified cells, **characterized in that** the cells are transduced with a vector according to Claims 1 to 5, bound to an agent specific to the surface marker, and separated from the genetically unmodified cells.

14. Method for the detection and analysis of cells, **characterized in that** the cells are transduced with a vector which contains a nucleic acid sequence coding for the surface marker CD34, a fragment of the same or a variant of the same, and the transduced cells are identified by detection of the surface marker, in which the cells do not naturally express CD34, a fragment or a variant of the same, wherein said fragment or variant is a truncated form of the CD34 surface antigen where protein kinase C (PKC) phosphorylation sites are deleted, or is a cytoplasmatically completely or partially deleted variant of the CD34 surface antigen.

15. Method for enriching cells which do not naturally express CD34, a fragment or a variant of the same, **characterized in that** the cells are transduced with a vector which contains a nucleic acid sequence coding for the surface marker CD34, a fragment of the same, or a variant of the same, and the transduced cells are bound to an agent specific to the surface marker, and separated from the cells which do not express the surface marker, wherein said fragment or variant is a truncated form of the CD34 surface antigen where protein kinase C (PKC) phosphorylation sites are deleted, or is a cytoplasmatically completely or partially deleted variant of the CD34 surface antigen.

16. Method according to Claim 14 or 15, **characterized in that** the nucleic acid sequence codes for a surface marker according to SEQ ID NO: 2 (CD34), 4 (tCD34) or 6 (dCD34) or for a fragment or a variant of the same, wherein the length of said fragment or variant lies between the length of tCD34 and dCD34.

17. Method according to Claims 14 or 15, **characterized in that** the nucleic acid sequence coding for the surface marker is the sequence indicated in SEQ ID NO: 1, 3 or 5 or a fragment, mutant or variant of the same, wherein the length of said nucleic acid encoding said fragment or variant lies between the lengths of the sequences indicated in SEQ ID NO:3 and 5.

18. Method according to Claims 14 to 17, **characterized in that** the vector is a retroviral vector.

19. Method according to Claims 14 to 18, **characterized in that** the vector corresponding to DSM 13396 is used.

20. Method according to Claims 12 to 19, **characterized in that** the cells are human cells.

21. Method according to Claims 20, **characterized in that** the cells are T-lymphocytes.

22. Kit for carrying out a method according to Claim 12, **characterized in that** it contains a vector according to Claims 1 to 5, means for the specific detection of the surface marker, and further agents and aids required for carrying out the detection.

23. Kit for carrying out a method according to Claim 14, **characterized in that** it contains a vector as mentioned in Claims 14 to 19, means for the specific detection of the surface marker and further agents and aids required for carrying out the detection.

24. Kit for carrying out a method according to Claim 13, **characterized in that** it contains a vector as mentioned in Claims 1 to 5, means for the specific binding of the surface marker and further agents and aids required for carrying out the detection.

25. Kit for carrying out a method according to Claim 15, **characterized in that** it contains a vector as mentioned in Claims 14 to 19, means for the specific binding of the surface marker and further agents and aids required for carrying out the detection.

26. Use of a vector according to Claims 1 to 5 for in vitro transduction of T-lymphocytes.

27. Use of a vector according to Claims 1 to 5 for the preparation of a pharmaceutical composition for gene therapeutic treatment.

28. Use of T-lymphocytes which are transduced with a vector according to Claims 1 to 5, for the preparation of a pharmaceutical composition for gene therapeutic treatment.

29. Use of a vector which contains a nucleic acid sequence coding for the surface marker CD34, a fragment of the same or a variant of the same, for enrichment, detection and analysis of cells *in* vitro that do not naturally express CD34, a fragment or a variant of the same, wherein said fragment or variant is a truncated form of the CD34 surface antigen where protein kinase C (PKC) phosphorylation sites are deleted, or is a cytoplasmatically completely or partially deleted variant of the CD34 surface antigen.

30. Use according to Claim 29, **characterized in that** the vector is a vector as mentioned in Claims 12 to 17.

31. Gene therapeutic drug, containing a vector according to Claims 1 to 5.

32. Gene therapeutic drug, containing T-lymphocytes, which are transduced with a vector according to Claims 1 to 5.

33. Use of a nucleic acid sequence (marker gene) coding for the CD34 surface antigen or a fragment of the same or a variant of the same (marker) for the detection of genetically modified cells, wherein said fragment or variant is a truncated form of the CD34 surface antigen where protein kinase C (PKC) phosphorylation sites are deleted, or is a cytoplasmatically completely or partially deleted variant of the CD34 surface antigen, **characterized in that** the nucleic acid sequence is incorporated into a gene transfer vector used for genetic modification, which contains a nucleic acid sequence (transgene) to be transferred into the cells, whereby the marker gene is chosen so that the marker is expressed on the surface of the cells to be transduced with the vector, whereby the transduced cells are identified by specific detection of the markers.

34. Use of a nucleic acid sequence (marker gene) coding for the CD34 surface antigen or a fragment of the same or a variant of the same (marker) for the detection of cells which do not naturally express CD34, a fragment or variant of the same, wherein said fragment or variant is a truncated form of the CD34 surface antigen where protein kinase C (PKC) phosphorylation sites are deleted, or is a cytoplasmatically completely or partially deleted variant of the CD34 surface antigen, **characterized in that** the nucleic acid sequence is incorporated into a vector, wherein the marker gene is chosen, so that the marker is expressed on the surface of the cells transduced with the vector, in which the transduced cells are identified by specific detection of the marker.

## Patentansprüche

1. Gentransfervektor, enthaltend
a) ein Transgen und
b) eine für einen Oberflächenmarker kodierende Nukleinsäuresequenz,
**dadurch gekennzeichnet, dass** der Oberflächenmarker das CD34-Oberflächenantigen oder ein Fragment desselben oder eine Variante desselben ist, wobei das Fragment oder die Variante eine trunkierte Form des CD34-Oberflächenantigens ist, in der Proteinkinase C (PKC) Phosphorylierungsstellen deletiert sind, oder eine zytoplasmatisch vollständig oder teilweise deletierte Variante des CD34-Oberflächenantigens ist.

2. Vektor nach Anspruch 1, **dadurch gekennzeichnet, dass** die Nukleinsäuresequenz für einen Oberflächenmarker in Übereinstimmung mit SEQ ID NO: 2 (CD34), 4 (tCD34) oder 6 (dCD34) oder für ein Fragmente oder eine Variante desselben kodiert, wobei die Länge des Fragments oder der Variante zwischen der Länge von tCD34 und dCD34 liegt.

3. Vektor nach den Ansprüchen 1 oder 2, **dadurch gekennzeichnet, dass** die für den Oberflächenmarker kodierende Nukleinsäuresequenz die in SEQ ID NO: 1, 3 oder 5 angegebene Sequenz oder ein Fragment oder eine Variante derselben ist, wobei die Länge der für das Fragment oder die Variante kodierenden Nukleinsäuresequenz zwischen den Längen der als SEQ ID NO: 3 und 5 angegebenen Sequenzen liegt.

4. Vektor nach den Ansprüchen 1 bis 3, **dadurch gekennzeichnet, dass** er ein retroviraler Vektor ist.

5. Vektor nach den Ansprüchen 1 bis 4, **dadurch gekennzeichnet, dass** er eine für einen weiteren Oberflächenmarker kodierende Nukleinsäuresequenz enthält.

6. Vektor mit der Zugangsnummer DSM 13396.

7. Vektor, **dadurch gekennzeichnet, dass** er eine für die Aminosäuresequenz nach SEQ ID NO: 6 kodierende Nukleinsäuresequenz enthält.

8. Vektor nach Anspruch 7, **dadurch gekennzeichnet, dass** er die Nukleinsäuresequenz nach SEQ ID NO: 5 enthält.

9. Wirtszelle, **dadurch gekennzeichnet, dass** sie mit einem Vektor nach den Ansprüchen 1 bis 8 transduziert ist.

10. Wirtszelle nach Anspruch 9, **dadurch gekennzeichnet, dass** sie eine menschliche Zelle ist.

11. Wirtszelle nach Anspruch 10, **dadurch gekennzeichnet, dass** sie ein T-Lymphozyt ist.

12. Verfahren zur Detektion genetisch veränderter Zellen, **dadurch gekennzeichnet, dass** die Zellen mit einem Vektor nach den Ansprüchen 1 bis 5 transduziert werden und die transduzierten Zellen durch Detektion des Oberflächenmarkers identifiziert werden.

13. Verfahren zur Selektion genetisch modifizierter Zellen, **dadurch gekennzeichnet, dass** die Zellen mit einem Vektor nach den Ansprüchen 1 bis 5 transduziert werden, mit einem für den Oberflächenmarker spezifischen Agens gebunden werden und von den genetisch nicht modifizierten Zellen getrennt werden.

14. Verfahren zur Detektion und Analyse von Zellen, **dadurch gekennzeichnet, dass** die Zellen mit einem Vektor transduziert werden, der eine für den Oberflächenmarker CD34, ein Fragment desselben oder eine Variante desselben kodierende Nukleinsäuresequenz enthält, und die transduzierten Zellen durch Detektion des Oberflächenmarkers identifiziert werden, wobei die Zellen nicht natürlicherweise CD34, ein Fragment oder eine Variante desselben exprimieren, wobei das Fragment oder die Variante eine trunkierte Form des CD34-Oberflächenantigens ist, bei der Proteinkinase C (PKC) Phosphorylierungsstellen deletiert sind, oder eine zytoplasmatisch vollständig oder teilweise deletierte Variante des CD34-Oberflächenantigens ist.

15. Verfahren zur Anreicherung von Zellen, welche nicht natürlicherweise CD34, ein Fragment oder eine Variante desselben exprimieren, **dadurch gekennzeichnet, dass** die Zellen mit einem Vektor transduziert werden, welcher eine für den Oberflächenmarker CD34, ein Fragment desselben oder eine Variante desselben kodierende Nukleinsäure enthält, und die transduzierten Zellen an ein für den Oberflächenmarker spezifisches Agens gebunden werden und von den Zellen getrennt werden, welche nicht den Oberflächenmarker exprimieren, wobei das Fragment oder die Variante eine trunkierte Form des CD34-Oberflächennantigens ist, in der Proteinkinase C (PKC) Phosphorylierungsstellen deletiert sind, oder eine zytoplasmatisch vollständig oder teilweise deletierte Variante des CD34-Oberflächenantigens ist.

16. Verfahren nach Anspruch 14 oder 15, **dadurch gekennzeichnet, dass** die Nukleinsäuresequenz für einen Oberflächenmarker nach SEQ ID NO: 2 (CD34), 4 (tCD34) oder 6 (dCD34) oder für ein Fragment oder eine Variante derselben kodiert, wobei die Länge des Fragments oder der Variante zwischen der Länge von tCD34 und dCD34 liegt.

17. Verfahren nach den Ansprüchen 14 oder 15, **dadurch gekennzeichnet, dass** die für den Oberflächenmarker kodierende Nukleinsäuresequenz die in SEQ ID NO: 1, 3 oder 5 angegebene Sequenz oder ein Fragment, eine Mutante oder eine Variante derselben ist, wobei die Länge der für das Fragment oder die Variante kodierenden Nukleinsäure zwischen den Längen der in SEQ ID NO: 3 und 5 angegebenen Nukleinsäuren liegt.

18. Verfahren nach den Ansprüchen 14 bis 17, **dadurch gekennzeichnet, dass** der Vektor ein retroviraler Vektor ist.

19. Verfahren nach den Ansprüchen 14 bis 18, **dadurch gekennzeichnet, dass** der DSM 13396 entsprechende Vektor verwendet wird.

20. Verfahren nach den Ansprüchen 12 bis 19, **dadurch gekennzeichnet, dass** die Zellen menschliche Zellen sind.

21. Verfahren nach Anspruch 20, **dadurch gekennzeichnet, dass** die Zellen T-Lymphozyten sind.

22. Kit zur Ausführung eines Verfahrens nach Anspruch 12, **dadurch gekennzeichnet, dass** es eine Vektor nach den Ansprüchen 1 bis 5, Mittel zur spezifischen Detektion des Oberflächenmarkers und weitere Agenzien und Hilfen enthält, die zur Ausführung der Detektion benötigt werden.

23. Kit zur Ausführung eines Verfahrens nach Anspruch 14, **dadurch gekennzeichnet, dass** es einen Vektor wie in den Ansprüchen 14 bis 19 erwähnt, Mittel zur spezifischen Detektion des Oberflächenmarkers und weitere Agenzien und Hilfen enthält, die zur Ausführung der Detektion benötigt werden.

24. Kit zur Ausführung eines Verfahrens nach Anspruch 13, **dadurch gekennzeichnet, dass** es einen Vektor wie in den Ansprüchen 1 bis 5 erwähnt, Mittel zur spezifischen Bindung des Oberflächenmarkers und weitere Agenzien und Hilfen enthält, die zur Ausführung der Detektion benötigt werden.

25. Kit zur Ausführung eines Verfahrens nach Anspruch 15, **dadurch gekennzeichnet, dass** es einen Vektor wie in den Ansprüchen 14 bis 19 erwähnt, Mittel zur spezifischen Bindung des Oberflächenmarkers und weitere Agenzien und Hilfen enthält, die zur Ausführung der Detektion benötigt werden.

26. Verwendung eines Vektors nach den Ansprüchen 1 bis 5 zur in *vitro*-Transduktion von T-Lymphozyten.

27. Verwendung eines Vektors nach den Ansprüchen 1 bis 5 zur Herstellung einer pharmazeutischen Zusammensetzung zur gentherapeutischen Behandlung.

28. Verwendung von mit einem Vektor nach den Ansprüchen 1 bis 5 transduzierten T-Lymphozyten zur Herstellung einer pharmazeutischen Zusammensetzung zur gentherapeutischen Behandlung.

29. Verwendung eines Vektors, welcher eine für den Oberflächenmarker CD34, ein Fragment desselben oder eine Variante desselben kodierende Nukleinsäuresequenz enthält, zur Anreicherung, Detektion und Analyse von Zellen *in vitro,* die nicht natürlicherweise CD34, ein Fragment oder eine Variante desselben exprimieren, wobei das Fragment oder die Variante eine trunkierte Form des CD34-Oberflächenantigens ist, bei der Proteinkinase C (PKC) Phosphorylierungsstellen deletiert sind, oder eine zytoplasmatisch vollständig oder teilweise deletierte Variante des CD34-Oberflächenantigens ist.

30. Verwendung nach Anspruch 29, **dadurch gekennzeichnet, dass** der Vektor ein Vektor wie in den Ansprüchen 12 bis 17 erwähnt ist.

31. Gentherapeutisches Medikament, enthaltend einen Vektor nach den Ansprüchen 1 bis 5.

32. Gentherapeutisches Medikament, enthaltend einen Vektor nach T-Lymphozyten, welche mit einem Vektor nach den Ansprüchen 1 bis 5 transduziert sind.

33. Verwendung einer für das CD34-Oberflächenantigen oder ein Fragment desselben oder eine Variante desselben (Marker) kodierenden Nukleinsäuresequenz (Markergen) zur Detektion von genetisch modifizierten Zellen, wobei das Fragment oder die Variante eine trunkierte Form des CD34-Oberflächenantigens ist, bei der Proteinkinase C (PKC) Phosphorylierungsstellen deletiert sind, oder eine zytoplasmatisch vollständig oder teilweise deletierte Variante des CD34-Oberflächenantigens ist, **dadurch gekennzeichnet, dass** die Nukleinsäuresequenz in einen zur genetischen Modifikation verwendeten Gentransfervektor eingebaut ist, welcher eine Nukleinsäuresequenz (Transgen) enthält, die in die Zellen transferiert werden soll, wobei das Markergen so ausgewählt wird, dass der Marker auf der Oberfläche der mit dem Vektor zu transduzierenden Zellen exprimiert wird, wobei die transduzierten Zellen durch spezifische Detektion der Marker identifiziert werden.

34. Verwendung einer für das CD34-Oberflächenantigen oder ein Fragment desselben oder eine Variante desselben (Marker) kodierenden Nukleinsäuresequenz (Markergen) zur Detektion von Zellen, welche nicht natürlicherweise CD34, ein Fragment oder eine Variante desselben exprimieren, wobei das Fragment oder die Variante eine trunkierte Form des CD34-Oberflächenantigens ist, bei der Proteinkinase C (PKC) Phosphorylierungsstellen deletiert sind, oder eine zytoplasmatisch vollständig oder teilweise deletierte Variante des CD34-Oberflächenantigens ist, **dadurch gekennzeichnet, dass** die Nukleinsäuresequenz in einen Vektor eingebaut ist, wobei das Markergen so ausgewählt ist, dass der Marker auf der Oberfläche der mit dem Vektor transduzierten Zellen exprimiert wird, wobei die transduzierten Zellen durch spezifische Detektion des Markers identifiziert werden.

## Revendications

1. Vecteur de transfert génique qui contient
(a) un transgène et
(b) une séquence d'acide nucléique codant un marqueur de surface,
**caractérisé en ce que** le marqueur de surface est l'antigène de surface CD34 ou un fragment de celui-ci ou un variant de celui-ci, ledit fragment ou variant étant une forme tronquée de l'antigène de surface CD34 où les sites de phosphorylation de protéine kinase C (PKC) sont délétés, ou est un variant de l'antigène de surface CD34 dont le domaine cytoplasmique est complètement ou partiellement délété.

2. Vecteur selon la revendication 1, **caractérisé en ce que** la séquence d'acide nucléique code un marqueur de surface conformément à SEQ ID NO : 2 (CD34), 4 (tCD34) ou 6 (dCD34) ou un fragment ou un variant de celui-ci, dans lequel la longueur dudit fragment ou variant se trouve entre la longueur de tCD34 et dCD34.

3. Vecteur selon la revendication 1 ou 2, **caractérisé en ce que** la séquence d'acide nucléique codant le marqueur de surface est la séquence indiquée dans SEQ ID NO : 1, 3 ou 5 ou un fragment ou un variant de celui-ci, la longueur dudit acide nucléique codant ledit fragment ou variant se trouvant entre les longueurs des séquences indiquées dans SEQ ID NO : 3 et 5.

4. Vecteur selon les revendications 1 à 3, **caractérisé en ce qu'**il est un vecteur rétroviral.

5. Vecteur selon les revendications 1 à 4, **caractérisé en ce qu'**il contient une séquence d'acide nucléique codant un autre marqueur de surface.

6. Vecteur ayant le numéro d'accès DSM 13396.

7. Vecteur **caractérisé en ce qu'**il contient une séquence d'acide nucléique codant la séquence d'acides aminés selon SEQ ID NO : 6.

8. Vecteur selon la revendication 7, **caractérisé en ce qu'**il contient la séquence d'acide nucléique selon SEQ ID NO : 5.

9. Cellule hôte, **caractérisée en ce qu'**elle est transduite avec un vecteur selon les revendications 1 à 8.

10. Cellule hôte selon la revendication 9, **caractérisée en ce qu'**elle est une cellule humaine.

11. Cellule hôte selon la revendication 10, **caractérisée en ce qu'**elle est un lymphocyte T.

12. Procédé pour la détection de cellules modifiées génétiquement, **caractérisé en ce que** les cellules sont transduites avec un vecteur selon les revendications 1 à 5 et les cellules transduites sont identifiées par détection du marqueur de surface.

13. Procédé pour la sélection de cellules modifiées génétiquement, **caractérisé en ce que** les cellules sont transduites avec un vecteur selon les revendications 1 à 5, liées à un agent spécifique du marqueur de surface et séparées des cellules non modifiées génétiquement.

14. Procédé pour la détection et l'analyse de cellules, **caractérisé en ce que** les cellules sont transduites avec un vecteur qui contient une séquence d'acide nucléique codant le marqueur de surface CD34, un fragment de celui-ci ou un variant de celui-ci, et les cellules transduites sont identifiées par détection du marqueur de surface, dans lequel les cellules n'expriment pas naturellement CD34, un fragment ou un variant de celui-ci, dans lequel ledit fragment ou variant est une forme tronquée de l'antigène de surface CD34 où les sites de phosphorylation de protéine kinase C (PKC) sont délétés, ou est un variant de l'antigène de surface CD34 dont le domaine cytoplasmique est complètement ou partiellement délété.

15. Procédé pour enrichir des cellules qui n'expriment pas naturellement CD34, un fragment ou un variant de celui-ci, **caractérisé en ce que** les cellules sont transduites avec un vecteur qui contient une séquence d'acide nucléique codant le marqueur de surface CD34, un fragment de celui-ci ou un variant de celui-ci, et les cellules transduites sont liées à un agent spécifique du marqueur de surface, et séparées des cellules qui n'expriment pas le marqueur de surface, dans lequel ledit fragment ou variant est une forme tronquée de l'antigène de surface CD34 où les sites de phosphorylation de protéine kinase C (PKC) sont délétés, ou est un variant de l'antigène de surface CD34 dont le domaine cytoplasmique est complètement ou partiellement délété.

16. Procédé selon la revendication 14 ou 15, **caractérisé en ce que** la séquence d'acide nucléique code un marqueur de surface selon SEQ ID NO : 2 (CD34), 4 (tCD34) ou 6 (dCD34) ou un fragment ou un variant de celui-ci, dans lequel la longueur dudit fragment ou variant se trouve entre la longueur de tCD34 et dCD34.

17. Procédé selon la revendication 14 ou 15, **caractérisé en ce que** la séquence d'acide nucléique codant le marqueur de surface est la séquence indiquée dans SEQ ID NO : 1, 3 ou 5 ou un fragment, mutant ou variant de celui-ci, dans lequel la longueur dudit acide nucléique codant ledit fragment ou variant se trouve entre les longueurs des séquences indiquées dans SEQ ID NO : 3 et 5.

18. Procédé selon les revendications 14 à 17, **caractérisé en ce que** le vecteur est un vecteur rétroviral.

19. Procédé selon les revendications 14 à 18, **caractérisé en ce que** le vecteur correspondant à DSM 13396 est utilisé.

20. Procédé selon les revendications 12 à 19, **caractérisé en ce que** les cellules sont des cellules humaines.

21. Procédé selon la revendication 20, **caractérisé en ce que** les cellules sont des lymphocytes T.

22. Kit pour réaliser un procédé selon la revendication 12, **caractérisé en ce qu'**il contient un vecteur selon les revendications 1 à 5, des moyens pour la détection spécifique du marqueur de surface et d'autres agents et auxiliaires requis pour réaliser la détection.

23. Kit pour réaliser un procédé selon la revendication 14, **caractérisé en ce qu'**il contient un vecteur tel que mentionné dans les revendications 14 à 19, des moyens pour la détection spécifique du marqueur de surface et d'autres agents et auxiliaires requis pour réaliser la détection.

24. Kit pour réaliser un procédé selon la revendication 13, **caractérisé en ce qu'**il contient un vecteur tel que mentionné dans les revendications 1 à 5, des moyens pour la liaison spécifique du marqueur de surface et d'autres agents et auxiliaires requis pour réaliser la détection.

25. Kit pour réaliser un procédé selon la revendication 15, **caractérisé en ce qu'**il contient un vecteur tel que mentionné dans les revendications 14 à 19, des moyens pour la liaison spécifique du marqueur de surface et d'autres agents et auxiliaires requis pour réaliser la détection.

26. Utilisation d'un vecteur selon les revendications 1 à 5 pour une transduction *in vitro* des lymphocytes T.

27. Utilisation d'un vecteur selon les revendications 1 à 5 pour la préparation d'une composition pharmaceutique pour un traitement par thérapie génique.

28. Utilisation de lymphocytes T qui sont transduits avec un vecteur selon les revendications 1 à 5, pour la préparation d'une composition pharmaceutique pour un traitement par thérapie génique.

29. Utilisation d'un vecteur qui contient une séquence d'acide nucléique codant le marqueur de surface CD34, un fragment de celui-ci ou un variant de celui-ci, pour l'enrichissement, la détection et l'analyse des cellules *in vitro* qui n'expriment pas naturellement CD34, un fragment ou un variant de celui-ci, dans laquelle ledit fragment ou variant est une forme tronquée de l'antigène de surface CD34 où les sites de phosphorylation de protéine kinase C (PKC) sont délétés, ou est un variant de l'antigène de surface CD34 dont le domaine cytoplasmique est complètement ou partiellement délété.

30. Utilisation selon la revendication 29, **caractérisée en ce que** le vecteur est un vecteur tel que mentionné dans les revendications 12 à 17.

31. Médicament thérapeutique génique, contenant un vecteur selon les revendications 1 à 5.

32. Médicament thérapeutique génique, contenant des lymphocytes T, qui sont transduits avec un vecteur selon les revendications 1 à 5.

33. Utilisation d'une séquence d'acide nucléique (gène marqueur) codant l'antigène de surface CD34 ou un fragment de celui-ci ou un variant de celui-ci (marqueur) pour la détection de cellules modifiées génétiquement, dans laquelle ledit fragment ou variant est une forme tronquée de l'antigène de surface CD34 où les sites de phosphorylation de protéines kinase C (PKC) sont délétés, ou est un variant de l'antigène de surface CD34 dont le domaine cytoplasmique est complètement ou partiellement délété, **caractérisée en ce que** la séquence d'acide nucléique est incorporée dans un vecteur de transfert génique utilisé pour une modification génétique, qui contient une séquence d'acide nucléique (transgène) à transférer dans les cellules, de sorte que le gène marqueur soit choisi de manière à ce que le marqueur soit exprimé sur la surface des cellules à transduire avec le vecteur, de sorte que les cellules transduites soient identifiées par détection spécifique des marqueurs.

34. Utilisation d'une séquence d'acide nucléique (gène marqueur) codant l'antigène de surface CD34 ou un fragment de celui-ci ou un variant de celui-ci (marqueur) pour la détection de cellules qui n'expriment pas naturellement CD34, un fragment ou un variant de celui-ci, dans laquelle ledit fragment ou variant est une forme tronquée de l'antigène de surface CD34 où les sites de phosphorylation de protéine kinase C (PKC) sont délétés, ou est un variant de l'antigène de surface CD34 dont le domaine cytoplasmique est complètement ou partiellement délété, **caractérisée en ce que** la séquences d'acide nucléique est incorporée dans un vecteur, dans laquelle le gène marqueur est choisi, de manière à ce que le marqueur soit exprimé sur la surface des cellules transduites avec le vecteur, dans laquelle les cellules transduites sont identifiées par détection spécifique du marqueur.
